# EUROPEAN PATENT APPLICATION

(11) **EP 4 850 789 A1**
(43) Date of publication of application: **09.12.2026**
(21) Application number: 25748174.7
(22) Date of filing: 28.01.2025
(51) Int. Cl.: B01D 53/38, B01D 53/44, C02F 3/10, C02F 3/34, C12M 1/00, C12N 1/00, C12N 1/20, C12N 11/02, C12N 11/14, C12N 11/084, C12N 11/087, C12N 11/093

(54) **METHOD FOR TREATING WASTE GAS OR WASTE LIQUID, AND DEVICE FOR TREATING WASTE GAS OR WASTE LIQUID**

(30) Priority: 29.01.2024 JP 2024010961
(71) Applicant: Toray Industries, Inc., Tokyo 103-8666 (JP)
(72) Inventor: KOMATSU, Shiomi, Kamakura-shi, Kanagawa 248-8555 (JP); KURIHARA, Hiroyuki, Kamakura-shi, Kanagawa 248-8555 (JP); HOE, Kazuki, Iyo-gun, Ehime 791-3193 (JP); YONEDA, Hirofumi, Iyo-gun, Ehime 791-3193 (JP); ISEKI, Katsuhiko, Otsu-shi, Shiga 520-8558 (JP)
(74) Representative: Kador & Partner Part mbB
(86) International application number: PCT/JP2025/002528
(87) International publication number: WO 2025/164592

(57) **Abstract**

Provided are an exhaust gas or exhaust liquid treatment method and an exhaust gas or exhaust liquid treatment apparatus which can efficiently decompose acrylonitrile.

An exhaust gas or exhaust liquid treatment method according to the present embodiment includes: a first step of bringing acrylonitrile in an exhaust gas or exhaust liquid into contact with a biocatalyst and a solvent activating a biocatalyst to convert the acrylonitrile into acrylamide and/or acrylate; and a second step of decomposing the acrylamide and/or acrylate.

## Description

### FIELD

The present embodiment relates to an exhaust gas or exhaust liquid treatment method, and an exhaust gas or exhaust liquid treatment apparatus, the exhaust gas or exhaust liquid containing acrylonitrile.

### BACKGROUND

Acrylonitrile is frequently used as a material for acrylic synthetic fibers and the like. On the other hand, acrylonitrile is highly toxic and is designated as a deleterious substance by law. Furthermore, acrylonitrile is volatile and also highly flammable. Therefore, when acrylonitrile is contained in an exhaust gas and/or exhaust liquid, it is necessary to recover and reuse the acrylonitrile from the exhaust gas and/or exhaust liquid, or to change the acrylonitrile into a safe substance.

### CITATION LIST

### PATENT LITERATURE

Patent Literature 1: WO 2015/19006 A

### NON PATENT LITERATURES

Non Patent Literature 1: "Removal of acrylonitrile vapor from waste gases by a trickle-bed air biofilter", Chungsying Lu et al., Bioresource Technology 75 (2000) 35-41
Non Patent Literature 2: "Evaluation of comprehensive wastewater treatment system for minimizing pollution load to environment", Koichi Eto (Research Project No. 04832020), 1993 Grants-in-Aid for Scientific Research (General Research (C)), Research Result Report, Yokohama National University Library
Non Patent Literature 3: "Biodegradation of high acrylamide concentrations in integrated fixed film activated sludge (IFAS) wastewater treatment system", Tongchai Sriwiriyarat et al., Biochemical Engineering Journal 159 (2020) 107566

### SUMMARY

### TECHNICAL PROBLEM

Such acrylonitrile is considered to be biodegraded using microorganisms. However, acrylonitrile is very volatile and is a hardly decomposable substance, and it is difficult to efficiently decompose acrylonitrile in a conventional biological treatment apparatus.

Therefore, the present embodiment is to solve the above problems, and provides an exhaust gas or exhaust liquid treatment method and an exhaust gas or exhaust liquid treatment apparatus which can efficiently treat acrylonitrile.

### SOLUTION TO PROBLEM

An exhaust gas or exhaust liquid treatment method according to the present embodiment includes: a first step of bringing acrylonitrile in an exhaust gas or exhaust liquid into contact with a biocatalyst and a solvent activating a biocatalyst to convert the acrylonitrile into acrylamide and/or acrylate; and a second step of decomposing the acrylamide and/or acrylate.

The biocatalyst is one or two or more biocatalysts selected from the group consisting of a microorganism and an enzyme having nitrile hydratase activity and a microorganism and an enzyme having nitrilase activity.

The microorganism having nitrile hydratase activity is one or two or more microorganisms selected from the genus Rhodococcus and the genus Comamonas.

The microorganism having nitrile hydratase activity is Rhodococcus rhodochrous.

The microorganism having nitrilase activity is one or two or more microorganisms selected from the genus Rhodococcus, the genus Acinetobacter, the genus Alcaligenes, and the genus Delftia.

The first step and the second step are steps independent from each other.

A reaction temperature in the first step is 20 to 30°C.

A pH of the acrylonitrile, the biocatalyst, and the solvent in the first step is a pH of 6 to 8.

An air flow rate to the acrylonitrile, the biocatalyst, and the solvent in the first step is 5 vvm or less.

A concentration of acrylonitrile in the exhaust gas or the exhaust liquid is 10000 ppm or less.

The first step is a step of causing the acrylonitrile and the solvent activating a biocatalyst to pass through the biocatalyst in a direction of gravity to convert the acrylonitrile and the solvent into a solution containing acrylamide and/or acrylate.

An exhaust gas or exhaust liquid treatment apparatus according to the present embodiment includes: an acrylonitrile converter including a casing that houses an immobilized biocatalyst, an acrylonitrile introduction port through which an exhaust gas or exhaust liquid containing acrylonitrile is introduced into the casing, a solvent introduction port through which a solvent activating the immobilized biocatalyst is introduced, and a drainage port through which a solution containing acrylamide and/or acrylate produced from acrylonitrile by the immobilized biocatalyst is discharged from the casing; and a treater treating the solution containing the acrylamide and/or the acrylate discharged from the acrylonitrile converter.

The solvent introduction port is the acrylonitrile introduction port.

The acrylonitrile converter is a fixed-bed catalyst reactor in which the casing is filled with the immobilized biocatalyst.

The acrylonitrile introduction port and the solvent introduction port are installed at an upper end of the casing.

The acrylonitrile converter includes an exhaust port through which the exhaust gas that has passed through the immobilized biocatalyst is exhausted from the casing.

A carrier of the immobilized biocatalyst is activated carbon or wood-based char.

The acrylonitrile converter further includes: a temperature sensor detecting a temperature of the immobilized biocatalyst; a temperature adjuster adjusting the temperature of the immobilized biocatalyst; and a temperature controller controlling the temperature adjuster based on a temperature measurement value from the temperature sensor.

The acrylonitrile converter further includes: a pH sensor detecting a pH of the immobilized biocatalyst; a pH adjuster introduction unit introducing a pH adjuster for adjusting the pH of the immobilized biocatalyst into the solvent; and a pH controller controlling the pH adjuster introduction unit based on a pH measurement value from the pH sensor.

The treater stores activated sludge for treating acrylamide and/or acrylate.

The activated sludge has amidase activity.

### BRIEF DESCRIPTION OF DRAWINGS

[Fig. 1] Fig. 1 is a block diagram illustrating a configuration example of an exhaust gas and/or exhaust liquid treatment apparatus according to a first embodiment.
[Fig. 2] Fig. 2 is a diagram illustrating a treatment example in the exhaust gas and/or exhaust liquid treatment apparatus according to the first embodiment.
[Fig. 3] Fig. 3 is a table showing characteristics of substances produced in each treatment stage of the exhaust gas and/or exhaust liquid treatment apparatus.
[Fig. 4] Fig. 4 is a block diagram illustrating a configuration example of an exhaust gas and/or exhaust liquid treatment apparatus according to a second embodiment.
[Fig. 5] Fig. 5 is a block diagram illustrating a configuration example of an exhaust gas and/or exhaust liquid treatment apparatus according to a third embodiment.
[Fig. 6A] Fig. 6A is a block diagram illustrating a configuration example of an exhaust gas and/or exhaust liquid treatment apparatus according to a fourth embodiment.
[Fig. 6B] Fig. 6B is a block diagram illustrating a configuration example of an exhaust liquid treatment apparatus according to a fifth embodiment.
[Fig. 7] Fig. 7 is a schematic diagram illustrating a configuration of a treatment apparatus according to Comparative Example 1.
[Fig. 8] Fig. 8 is a schematic diagram illustrating a configuration of a treatment apparatus according to Comparative Examples 2 and 3.
[Fig. 9] Fig. 9 is a table showing comparison results of Comparative Examples 1 to 3 and Examples 1 to 3.

### DESCRIPTION OF EMBODIMENTS

Hereinafter, embodiments according to the present invention are described with reference to the drawings. The present embodiment does not limit the present invention. The drawings are schematic or conceptual, and a ratio between portions and the like is not necessarily the same as actual ones. In the specification and the drawings, elements similar to those described above with respect to the previously described drawings are denoted by the same reference numerals, and the detailed description thereof is appropriately omitted.

### (First Embodiment)

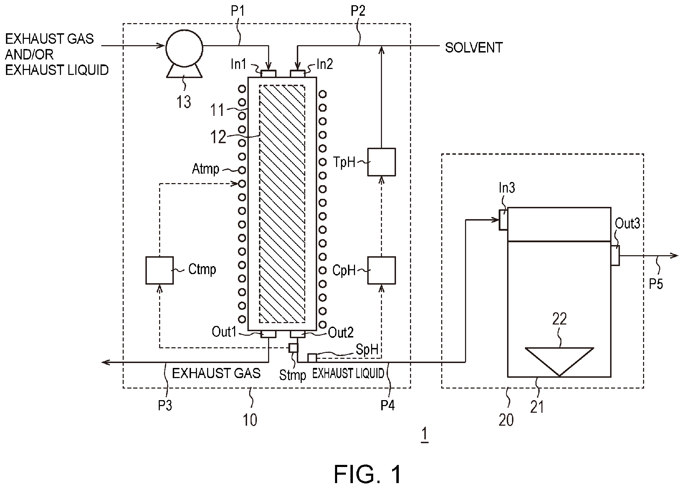

Fig. 1 is a block diagram illustrating a configuration example of an exhaust gas and/or exhaust liquid treatment apparatus according to a first embodiment. An exhaust gas and/or exhaust liquid treatment apparatus 1 includes an acrylonitrile converter 10 and a solvent treater 20. Note that, in Fig. 1, a solid arrow indicates a pipe and a flow of a liquid, or a gas. Dashed arrows indicate signal flows.

The acrylonitrile converter 10 includes a casing 11, a first introduction port In1, a second introduction port In2, an exhaust port Out1, a drainage port Out2, a biocatalyst 12, and a pump 13. The acrylonitrile converter 10 is a fixed-bed catalyst reactor in which the biocatalyst 12 is filled in the casing 11.

The casing 11 has, for example, a hollow cylindrical shape, and is a sealed container so that acrylonitrile does not leak to the outside. The casing 11 is made of, for example, a metal material such as stainless steel (SUS) having resistance to acrylonitrile, or a resin material such as hard polyvinyl chloride. The biocatalyst 12 is housed in the casing 11. The casing 11 is airtightly configured so that volatile acrylonitrile does not leak to the outside. For example, the air flow rate of the casing 11 is preferably 5 vvm (gas Volume per liquid Volume per Minute, liquid is the amount of liquid retained by the carrier, and is approximately regarded as the carrier volume in the present invention) or less, and more preferably 0.5 vvm or less. The reason for this is to prevent volatile acrylonitrile from leaking to the outside of the casing 11. When the air flow rate is too high, the pressure applied to the container increases, which increases the risk of acrylonitrile leakage. Furthermore, when the air flow rate is too high, the carrier passing rate of acrylonitrile exceeds the decomposition rate, and the amount of acrylonitrile discharged in an unreacted state increases. The concentration of acrylonitrile in the gas or exhaust liquid is preferably 10000 ppm or less, and more preferably 5000 ppm or less. This is because when the concentration of acrylonitrile exceeds 10000 ppm, the concentration of acrylamide and acrylate decomposed by the acrylonitrile converter also increases, so that the load in the decomposition in step 2 increases. Note that the lower limit of the concentration of acrylonitrile in the gas or exhaust liquid is not particularly limited, but is preferably 1 ppm or more, and more preferably 10 ppm or more.

The biocatalyst 12 is, for example, an immobilized biocatalyst in which a microorganism having nitrile hydratase activity or an enzyme having nitrile hydratase activity is immobilized on a carrier. Alternatively, the biocatalyst 12 is, for example, a catalyst in which a microorganism having nitrilase activity or an enzyme having nitrilase activity is immobilized on a carrier. That is, the biocatalyst 12 is one or two or more biocatalysts selected from the group consisting of a microorganism and an enzyme having nitrile hydratase activity and a microorganism and an enzyme having nitrilase activity. The carrier may be made of, for example, polyurethane, activated carbon, wood-based char, glass beads, silica gel, polyacrylamide, polyvinyl alcohol, carrageenan, alginic acid, agar, gelatin, a resin tube, or the like. The microorganism having nitrile hydratase activity is, for example, one or two or more microorganisms selected from the genus Rhodococcus, the genus Comamonas, the genus Pseudomonas, the genus Arthrobacter, the genus Brevibacterium, and the genus Streptomyces, and more preferably Rhodococcus rhodochrous. The enzyme is, for example, an enzyme having nitrile hydratase activity produced by a bacterium belonging to the genus Rhodococcus, more preferably Rhodococcus rhodochrous. The microorganism or enzyme having nitrile hydratase activity can more efficiently change acrylonitrile to acrylamide by a hydration reaction as compared with activated sludge. The microorganism having nitrilase activity is, for example, one or two or more microorganisms selected from the genus Rhodococcus, the genus Acinetobacter, the genus Alcaligenes, the genus Delftia, the genus Pseudomonas, the genus Comamonas, the genus Bacillus, the genus Arthrobacter, the genus Fusarium, and the genus Klebsiella, and more preferably the genus Rhodococcus, the genus Acinetobacter, the genus Alcaligenes, or the genus Delftia. The microorganism or enzyme having nitrilase activity can directly convert acrylonitrile into acrylates.

The exhaust gas and/or exhaust liquid containing acrylonitrile is introduced into the casing 11 through first introduction port (acrylonitrile introduction port) In1. The first introduction port In1 is provided on one end side (for example, the upper end) of the casing 11, and is connected to the pump 13 through a pipe P1. The pump 13 and the first introduction port In1 communicate with each other in an airtight state by the pipe P1. The pipe P1 sends the exhaust gas and/or exhaust liquid from the pump 13 to the first introduction port In1. The pump 13 sends the exhaust gas and/or exhaust liquid from the outside into the casing 11 via the pipe P1.

A solvent activating the microorganism or enzyme is introduced into the casing 11 through the second introduction port (solvent introduction port) In2, and this solvent is supplied to the biocatalyst 12. The second introduction port In2 is provided on one end side (for example, the upper end) of the casing 11, and is connected to a pipe P2. The second introduction port In2 communicates with the outside in an airtight state by the pipe P2, and the solvent from the outside is fed to the second introduction port In2 by the pipe P2. The pipe P2 feeds the solvent from the outside to the second introduction port In2. The solvent may be, for example, water. Water is utilized in a hydration reaction of acrylonitrile with the microorganism and an enzyme having nitrile hydratase activity when acrylamide is produced. Water is utilized in a hydration reaction of acrylonitrile with the microorganism and an enzyme having nitrilase activity when acrylate is produced. Examples of water include pure water and an aqueous solution in which an acid, a salt, or the like is dissolved in water. Examples of the acid include phosphoric acid, acetic acid, citric acid, boric acid, acrylic acid, and formic acid. Examples of the salt include a sodium salt, a potassium salt, and an ammonium salt of the above-described acids. Specific examples of water include, but are not particularly limited to, water such as pure water, ultrapure water, and city water, and buffers such as a Tris buffer, a phosphate buffer, an acetate buffer, a citrate buffer, and a borate buffer. The pH (20°C) of the raw material water is 5 to 9, and is preferably close to neutral. The pH of the mixture of acrylonitrile, the biocatalyst 12, and the solvent is preferably a pH of 6 to 8. This is because the proliferation of microorganisms is vigorous and the enzyme activity is high. The reaction temperature of the mixture of acrylonitrile, the biocatalyst 12, and the solvent is preferably 20°C to 30°C. This is because the proliferation of microorganisms is vigorous and the enzyme activity is high. The introduction port In2 may not be provided when a solvent is already present in the exhaust liquid or a necessary component is added in advance at the time of treating the exhaust liquid containing acrylonitrile.

The introduction ports In1 and In2 may be shared. That is, the acrylonitrile converter 10 may have a single introduction port, and the exhaust liquid (exhaust gas) and the solvent may be introduced from the single introduction port.

The exhaust gas that has passed through the biocatalyst 12 is exhausted from the casing 11 through the exhaust port Out1. The exhaust port Out1 is provided on the other end side (for example, the lower end) of the casing 11, and is connected to a pipe P3. The exhaust port Out1 communicates with the outside in an airtight state by the pipe P3, and sends the exhaust gas that has passed through the biocatalyst 12 to the outside. The exhaust gas from the exhaust port Out1 may be released to the atmosphere depending on the residual amount of acrylonitrile contained in the exhaust gas. Alternatively, the exhaust gas from the exhaust port Out1 may be introduced into the first introduction port In1 of the acrylonitrile converter 10 again, or may be further introduced into another exhaust gas converter and treated. When only the exhaust liquid containing acrylonitrile is treated, the exhaust port Out1 may not be provided.

The solvent supplied to the biocatalyst 12 of the casing 11 is exhausted from the casing 11 through the drainage port Out2. The acrylonitrile in the exhaust gas and/or exhaust liquid is converted to acrylamide by a microorganism or enzyme having nitrile hydratase activity when the acrylonitrile passes through the biocatalyst 12 and is brought into contact with the biocatalyst 12. Alternatively, the acrylonitrile in the exhaust gas and/or exhaust liquid is converted to acrylate by a microorganism or enzyme having nitrilase activity when the acrylonitrile passes through the biocatalyst 12 and is brought into contact with the biocatalyst 12. The solvent supplied to the biocatalyst 12 activates a microorganism or enzyme and dissolves acrylamide or acrylate. Therefore, the solvent to be exhausted from the drainage port Out2 of the casing 11 contains acrylamide or acrylate.

The solvent to be exhausted from the drainage port Out2 is fed to the solvent treater 20 via a pipe P4 while containing acrylamide or acrylate. The drainage port Out2 is provided on the other end side (for example, the lower end) of the casing 11, and is connected to the pipe P4. The drainage port Out2 communicates with the solvent treater 20 in an airtight state by the pipe P4, and the solvent containing acrylamide or acrylate supplied to the biocatalyst 12 is fed to the solvent treater 20 through the drainage port Out2.

The acrylonitrile converter 10 is preferably disposed so that the first and second introduction ports In1 and In2 are arranged upward (in a direction opposite to the direction of gravity) and the exhaust port Out1 and the drainage port Out2 are arranged downward (in the direction of gravity). Thereby, the acrylonitrile converter 10 can cause the acrylonitrile and the solvent activating a biocatalyst to pass through the biocatalyst 12 in the direction of gravity and smoothly convert the acrylonitrile and the solvent into a solution containing acrylamide and/or acrylate.

The acrylonitrile converter 10 further includes a temperature sensor Stmp, a temperature controller Ctmp, a temperature adjuster Atmp, a pH sensor SpH, a pH controller CpH, and a pH adjuster introduction unit TpH.

The temperature sensor Stmp is provided in the pipe P4 near the drainage port Out2, and detects the temperature of the solvent passing through the pipe P4. The solvent temperature immediately after drainage from the casing 11 is substantially equal to the temperature of the biocatalyst 12. Therefore, it can be said that the temperature sensor Stmp detects the temperature of the biocatalyst 12.

The temperature controller Ctmp controls the temperature adjuster Atmp based on a temperature measurement value from the temperature sensor Stmp. For example, when the temperature of the biocatalyst 12 is maintained at room temperature (about 25 degrees to about 30 degrees), the temperature controller Ctmp controls the temperature adjuster Atmp to be turned on or off so that the temperature measurement value from the temperature sensor Stmp is equal to room temperature (about 25 degrees to about 30 degrees).

The temperature adjuster Atmp is provided outside or inside the casing 11 in order to adjust the temperature of the biocatalyst 12. For example, the temperature adjuster Atmp is a heater that increases the temperature of the biocatalyst 12 or a cooler that decreases the temperature of the biocatalyst 12. When the temperature adjuster Atmp is a heater, the temperature adjuster Atmp may be, for example, an electric heating wire or the like. When the temperature adjuster Atmp is a cooler, the temperature adjuster Atmp may be, for example, a Peltier element or the like. The temperature adjuster Atmp electrically increases or decreases the temperature of the biocatalyst 12 under the control of the temperature controller Ctmp, and adjusts the temperature of the biocatalyst 12 within a desired range (for example, 20°C to 30°C). The temperature adjuster Atmp may be provided outside or inside the pipe P2 or may be provided outside or inside a solvent tank in order to adjust the temperature of the solvent to be injected into the casing.

The pH sensor SpH is provided in the pipe P4 near the drainage port Out2, and detects the pH of the solvent passing through the pipe P4. The solvent exhausted from the casing 11 has a pH approximately equal to the pH of the biocatalyst 12. Therefore, it can be said that the pH sensor SpH detects the pH of the biocatalyst 12.

The pH controller CpH controls the pH adjuster introduction unit TpH based on a pH measurement value from the pH sensor SpH. For example, when the pH of the biocatalyst 12 is maintained in a desired range (for example, pH = 5 to 9), the pH controller CpH controls the pH adjuster introduction unit TpH to be turned on or off so that the pH measurement value from the pH sensor SpH falls within a desired range (for example, pH = 5 to 9, and preferably pH = 6 to 8 as a range in which the proliferation of microorganisms is vigorous and the enzyme activity is high).

The pH adjuster introduction unit stores a pH adjuster that adjusts the pH of the biocatalyst 12, and introduces the pH adjuster into the solvent, which is scheduled to be introduced into the casing 11, in the pipe P2. The pH adjuster is, for example, hydrochloric acid, sulfuric acid, phosphoric acid, nitric acid, or the like in the case of lowering the pH, or sodium hydroxide, potassium hydroxide, calcium hydroxide, ammonia, or the like in the case of raising the pH.

The solvent treater 20 includes a container 21, a gas supply unit 22, a third introduction port In3, and a drainage port Out3.

The container 21 receives the solvent from the exhaust gas and/or exhaust liquid converter 10 and stores activated sludge for treating acrylamide or acrylate contained in the solvent. The activated sludge preferably has amidase activity, and acrylamide is changed to acrylate or the like by the amidase activity. The activated sludge further decomposes the acrylate and converts it into other harmless organic substances or inorganic substances. As a result, acrylamide is detoxified and can be released to the outside. Note that, when a microorganism and an enzyme having nitrilase activity are used as the biocatalyst 12, acrylonitrile is directly converted into acrylate, so that it is sufficient that the activated sludge converts the acrylate into a harmless organic substance or inorganic substance. Therefore, the treatment of acrylonitrile is relatively simple and short.

The solvent from the acrylonitrile converter 10 is introduced through the third introduction port In3 via the pipe P4, and this solvent is supplied into the container 21. The third introduction port In3 is provided on one end side (for example, the upper end) of the casing 11, and is connected to the pipe P4. The pipe P4 feeds the solvent from the exhaust gas and/or exhaust liquid converter 10 to the third introduction port In3.

The gas supply unit 22 supplies a gas (for example, air) as bubbles to the activated sludge in the container 21. As a result, the gas supply unit 22 aerates the activated sludge in the container 21. The activated sludge can maintain its active state for a long time by being aerated, and can efficiently change acrylamide to acrylate or change acrylate to a harmless organic substance or inorganic substance.

The drainage port Out3 is provided in the upper portion of the container 21, and is connected to a pipe P5. The acrylate treated with the activated sludge in the container 21, a harmless organic substance or inorganic substance, or the like is discharged to the outside through the drainage port Out3 by the pipe P5. For example, the drainage port Out3 is used to transfer a liquid treated with activated sludge to a facility for separating activated sludge components via the pipe P5, or to discharge a supernatant liquid generated by temporarily stopping aeration and precipitating activated sludge to the outside via the pipe P5. Since the activated sludge is aerated, it is preferable that the container 21 is not a sealed container, and has an opened upper side or an open portion.

The liquid discharged from the drainage port Out3 may be discharged to a river or the like after being further subjected to a treatment such as disinfection.

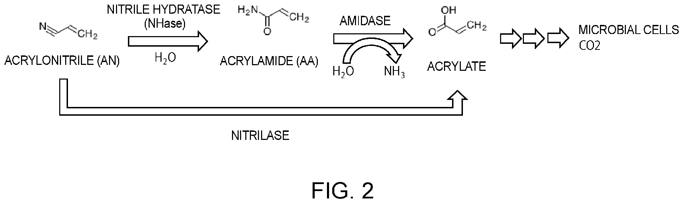

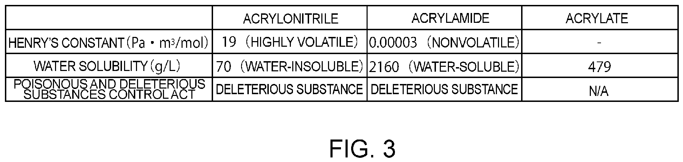

Fig. 2 is a diagram illustrating a treatment example in the exhaust gas and/or exhaust liquid treatment apparatus according to the first embodiment. Fig. 3 is a table showing characteristics of substances produced in each treatment stage of the exhaust gas and/or exhaust liquid treatment apparatus.

As illustrated in Fig. 2, an exhaust gas and/or exhaust liquid containing acrylonitrile (AN) is introduced into the acrylonitrile converter 10, and is changed to acrylamide (AA) by a hydration reaction by a microorganism or enzyme, which is immobilized on the biocatalyst 12 in the acrylonitrile converter 10, having nitrile hydratase activity (first step). Referring to Fig. 3, it can be seen that acrylonitrile has a relatively high Henry's constant and high volatility. Acrylonitrile has relatively low water solubility and is water-insoluble. Furthermore, acrylonitrile is designated as a deleterious substance by Poisonous and Deleterious Substances Control Act. That is, acrylonitrile is a volatile and water-insoluble deleterious substance. For this reason, acrylonitrile needs to be converted into acrylamide in a sealed atmosphere so as not to leak to the outside. The acrylonitrile converter 10 according to the present embodiment brings the exhaust gas and/or exhaust liquid into contact with the biocatalyst 12 in the hermetically sealed and airtight casing 11. Therefore, acrylonitrile can be converted into acrylamide in a sealed atmosphere without leaking to the outside.

On the other hand, as shown in Fig. 3, acrylamide is much lower in Henry's constant than acrylonitrile and much higher in water solubility than acrylonitrile. Thus, although acrylamide is designated as a deleterious substance by Poisonous and Deleterious Substances Control Act, acrylamide is nonvolatile and water-soluble. Thus, the solvent (for example, water) can easily dissolve acrylamide without dissolving acrylonitrile, and can move from the acrylonitrile converter 10 to the solvent treater 20 in a state of being separated from acrylonitrile, as a solvent containing acrylamide. The solvent containing acrylamide is aerated in the activated sludge in the container 21 of the opened solvent treater 20. However, since acrylamide is nonvolatile and water-soluble, acrylamide hardly volatilizes and leaks to the outside. Thus, the solvent treater 20 can safely aerate acrylamide, which is a deleterious substance, in the activated sludge in the opened container 21. Thereby, the solvent treater 20 treats the acrylamide of the solvent exhausted from the acrylonitrile converter 10 (second step).

As illustrated in Fig. 2, acrylamide produces acrylate and ammonia in a hydration reaction by activated sludge having amidase activity.

As shown in Fig. 3, the acrylate is not designated as a deleterious substance in Poisonous and Deleterious Substances Control Act, and has lower toxicity than acrylonitrile and acrylamide. However, from the viewpoint of toxicity to aquatic organisms, it is preferable that the acrylate is contained as little as possible in the liquid to be released into the environment.

When the biocatalyst 12 is a microorganism and an enzyme having nitrilase activity, acrylonitrile is directly converted into acrylate in the acrylonitrile converter 10.

As described above, in the exhaust gas and/or exhaust liquid treatment apparatus 1 according to the present embodiment, acrylonitrile can be safely converted into acrylamide or acrylate in a sealed state in the acrylonitrile converter 10 (first step), and subsequently, acrylamide can be efficiently converted into acrylate or the like having low toxicity by aeration in the solvent treater 20, and the acrylate can be further converted into a harmless organic substance or inorganic substance (second step). As a result, according to the present embodiment, acrylonitrile can be efficiently decomposed and treated.

Note that the first step and the second step are preferably steps independent from each other in order to safely treat volatile acrylonitrile in a sealed state and efficiently treat acrylamide and/or acrylate by aeration.

### (Second Embodiment)

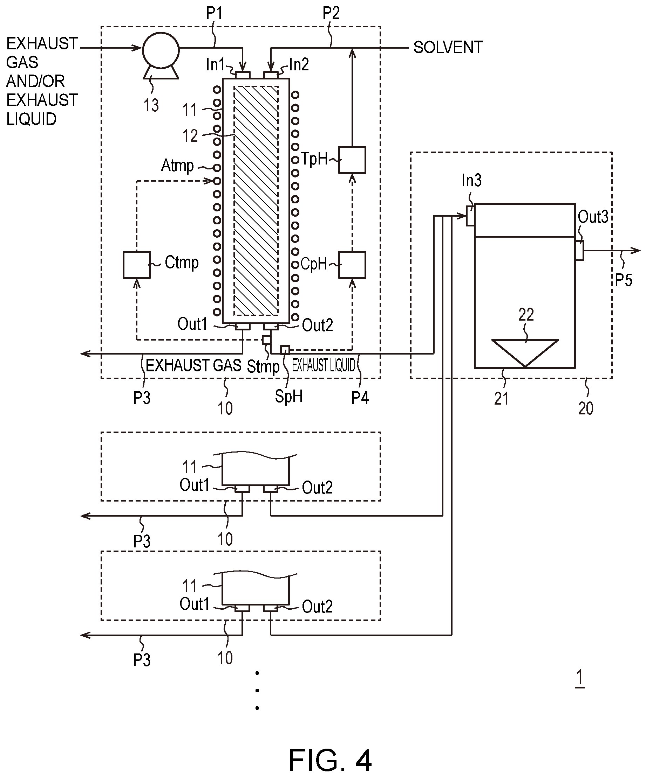

Fig. 4 is a block diagram illustrating a configuration example of an exhaust gas and/or exhaust liquid treatment apparatus according to a second embodiment. The exhaust gas and/or exhaust liquid treatment apparatus 1 according to the second embodiment includes a plurality of acrylonitrile converters 10. The drainage ports Out2 of the plurality of acrylonitrile converters 10 are commonly connected to the third introduction port In3 of one solvent treater 20. That is, the plurality of acrylonitrile converters 10 are connected in parallel to one solvent treater 20. The internal configuration of each acrylonitrile converter 10 may be the same as that of the acrylonitrile converter 10 according to the first embodiment.

The solvent treater 20 introduces solvents from the plurality of acrylonitrile converters 10 and is aerated in the activated sludge. Thus, the solvent treater 20 can further decompose acrylamide or acrylate contained in a large amount of solvent by microbial metabolism.

When the casing 11 and the biocatalyst 12 of the acrylonitrile converter 10 are elongated, microorganisms and enzymes are easily deactivated. On the other hand, in the second embodiment, by connecting the plurality of acrylonitrile converters 10 parallel to one solvent treater 20, the lengths of the casing 11 and the biocatalyst 12 of each acrylonitrile converter 10 can be shortened. This makes it possible to maintain the active state of microorganisms and enzymes for a long time.

Other configurations of the second embodiment may be similar to the corresponding configurations of the first embodiment. Therefore, the second embodiment can also obtain the same effects as those of the first embodiment.

### (Third Embodiment)

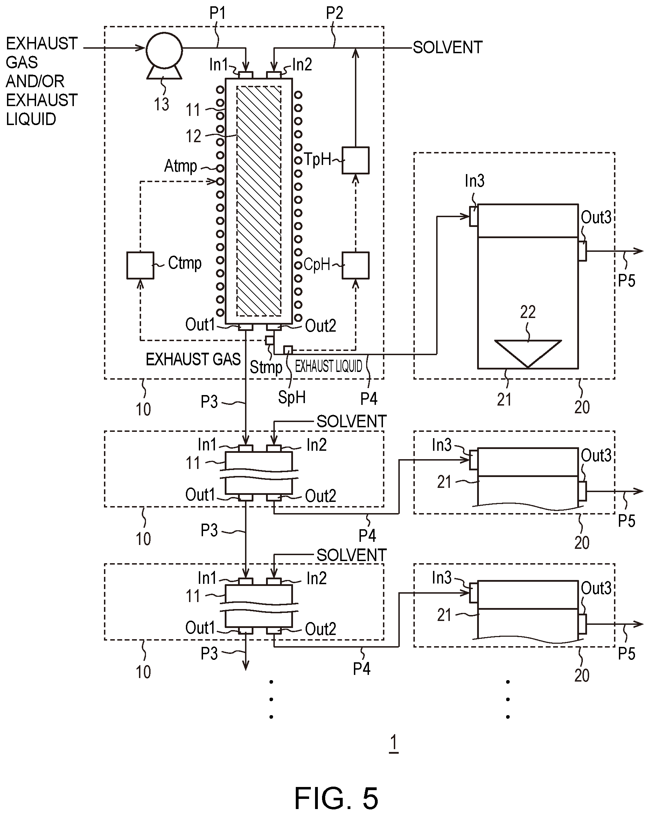

Fig. 5 is a block diagram illustrating a configuration example of an exhaust gas and/or exhaust liquid treatment apparatus according to a third embodiment. The exhaust gas and/or exhaust liquid treatment apparatus 1 according to the third embodiment includes a plurality of acrylonitrile converters 10 and a plurality of solvent treaters 20. The plurality of acrylonitrile converters 10 correspond to the plurality of solvent treaters 20. The drainage ports Out2 of the plurality of acrylonitrile converters 10 are connected to the third introduction port In3 of the corresponding solvent treaters 20.

The exhaust port Out1 of the first acrylonitrile converter 10 among the plurality of acrylonitrile converters 10 is connected to the first introduction port In1 of the subsequent second acrylonitrile converter 10 via the pipe P3. That is, the plurality of acrylonitrile converters 10 are connected in series via the exhaust port Out1 and the first introduction port In1. The exhaust gas exhausted from the first acrylonitrile converter 10 is treated again in the second acrylonitrile converter 10. As a result, even when acrylonitrile remains in the exhaust gas exhausted from the first acrylonitrile converter 10, the acrylonitrile can be converted into acrylamide or acrylate in the second acrylonitrile converter 10. As such, the plurality of acrylonitrile converters 10 can treat the exhaust gas in succession to ensure that a larger amount of acrylonitrile is converted from the exhaust gas into acrylamide or acrylate. Note that the number of the plurality of acrylonitrile converters 10 connected in series (continuously) may be 3 or more.

When the casing 11 and the biocatalyst 12 of the exhaust gas converter 10 are elongated, microorganisms and enzymes are easily deactivated. On the other hand, in the third embodiment, by connecting the plurality of acrylonitrile converters 10 in series, the lengths of the casing 11 and the biocatalyst 12 of each acrylonitrile converter 10 can be shortened. This makes it possible to maintain the active state of microorganisms and enzymes for a long time.

Other configurations of the third embodiment may be similar to the corresponding configurations of the first embodiment. Therefore, the third embodiment can also obtain the same effects as those of the first embodiment.

### (Fourth Embodiment)

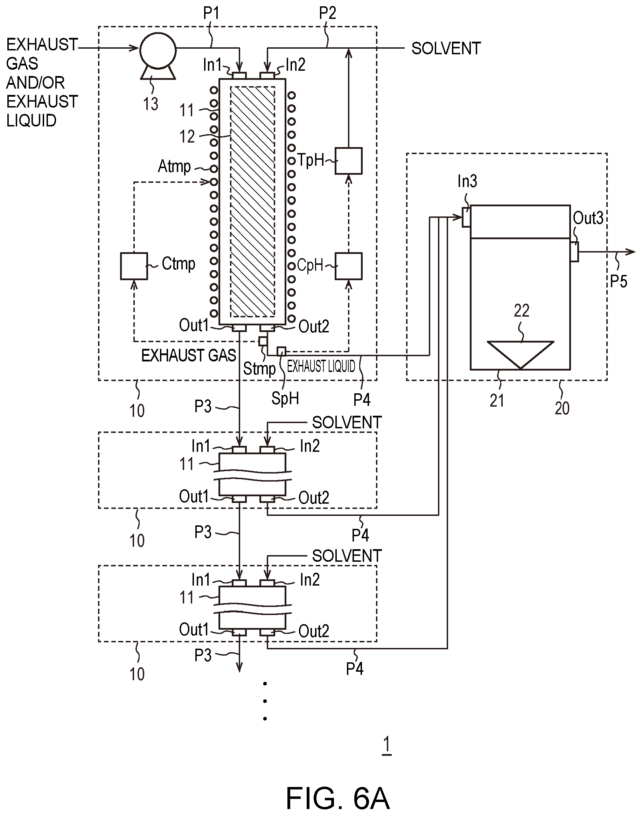

Fig. 6A is a block diagram illustrating a configuration example of an exhaust gas and/or exhaust liquid treatment apparatus according to a fourth embodiment. The exhaust gas and/or exhaust liquid treatment apparatus 1 according to the fourth embodiment is a combination of the second and third embodiments. Therefore, the exhaust gas and/or exhaust liquid treatment apparatus 1 includes the plurality of acrylonitrile converters 10, and the drainage ports Out2 of the plurality of acrylonitrile converters 10 are commonly connected to the third introduction port In3 of one solvent treater 20. That is, the plurality of acrylonitrile converters 10 are connected in parallel to one solvent treater 20.

Furthermore, the exhaust port Out1 of the first acrylonitrile converter 10 among the plurality of acrylonitrile converters 10 is connected to the first introduction port In1 of the subsequent second acrylonitrile converter 10 via the pipe P3. That is, the plurality of acrylonitrile converters 10 are connected in series via the exhaust port Out1 and the first introduction port In1. Note that the number of the plurality of acrylonitrile converters 10 connected in series (continuously) may be 3 or more.

Other configurations of the fourth embodiment may be similar to the corresponding configurations of the second or third embodiment. Therefore, the fourth embodiment can also obtain the same effects as those of the second and third embodiments.

### (Fifth Embodiment)

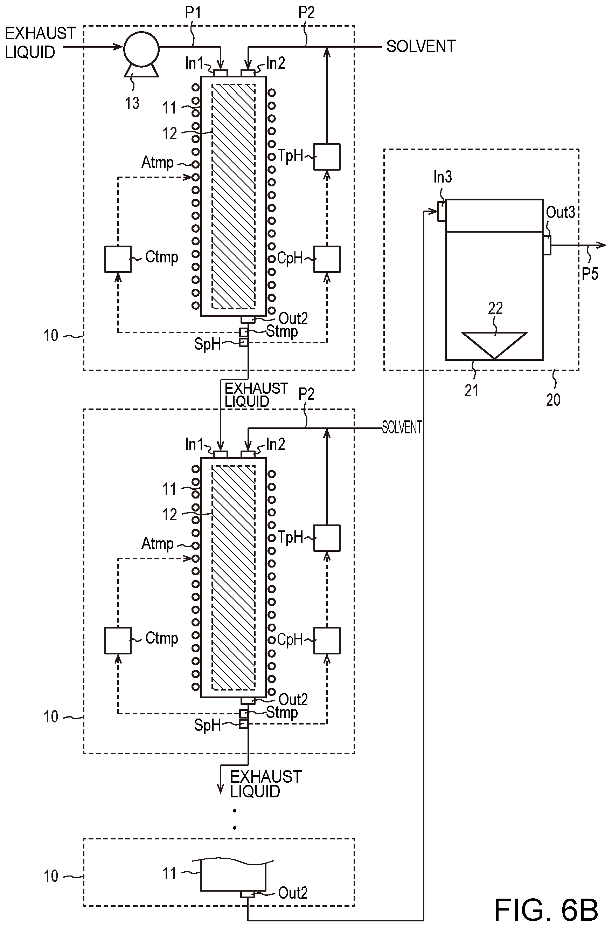

Fig. 6B is a block diagram illustrating a configuration example of an exhaust liquid treatment apparatus according to a fifth embodiment. The exhaust liquid treatment apparatus 1 according to the fifth embodiment includes a plurality of acrylonitrile converters 10 and one solvent treater 20. The drainage port Out2 of the first acrylonitrile converter 10 among the plurality of acrylonitrile converters 10 is connected to the first introduction port In1 of the second acrylonitrile converter 10 of the next stage via the pipe P3. That is, the plurality of acrylonitrile converters 10 are connected in series via the drainage port Out2 and the first introduction port In1. The exhaust liquid discharged from the first acrylonitrile converter 10 is treated again in the second acrylonitrile converter 10. As a result, even when acrylonitrile remains in the exhaust liquid discharged from the first acrylonitrile converter 10, the acrylonitrile can be converted into acrylamide or acrylate in the second acrylonitrile converter 10. As such, the plurality of acrylonitrile converters 10 can treat the exhaust liquid in succession to ensure that a larger amount of acrylonitrile is converted from the exhaust liquid into acrylamide or acrylate. Note that the number of the plurality of acrylonitrile converters 10 connected in series (continuously) may be 3 or more.

The drainage port Out2 of the last acrylonitrile converter 10 is connected to the third introduction port In3 of one solvent treater 20. That is, the plurality of acrylonitrile converters 10 are connected in series to one solvent treater 20. The internal configuration of each acrylonitrile converter 10 may be the same as that of the acrylonitrile converter 10 according to the first embodiment, but the exhaust port Out1 may not be provided.

### EXAMPLES

Hereinafter, the present invention will be specifically described with reference to Comparative Examples and Examples.

The acrylonitrile-containing exhaust gas was prepared by volatilizing acrylonitrile by ventilation to an aqueous acrylonitrile solution, and supplied to each treatment apparatus of Comparative Examples and Examples. Specifically, 50 mL of a 2 wt% aqueous solution of acrylonitrile was put in a sealed glass bottle, and ventilation was performed through a tube inserted into the aqueous solution of acrylonitrile to volatilize the acrylonitrile. The glass bottle was connected to each treatment apparatus through a Pharmed tube so that the gas phase portion in the glass bottle flows into each treatment apparatus.

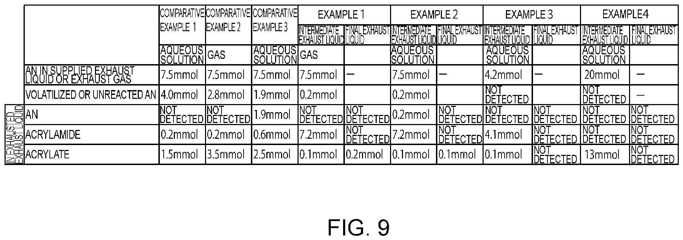

Note that the amount of acrylonitrile in the supplied exhaust gas shown in Fig. 9 is a numerical value calculated from the amount of change in the amount of acrylonitrile in the aqueous acrylonitrile solution before and after ventilation, and the amount of acrylonitrile in the aqueous acrylonitrile solution is a numerical value detected by high performance liquid chromatography. The amount of acrylonitrile in the exhaust liquid, and the amount of acrylonitrile, the amount of acrylamide, and the amount of acrylate in the discharged exhaust liquid are numerical values detected by high performance liquid chromatography, and the amount of volatilized or unreacted acrylonitrile is a numerical value calculated by subtracting the amounts of acrylamide and acrylate in the discharged exhaust liquid from the amounts of acrylonitrile in the supplied exhaust gas and the exhaust liquid.

### (Comparative Example 1)

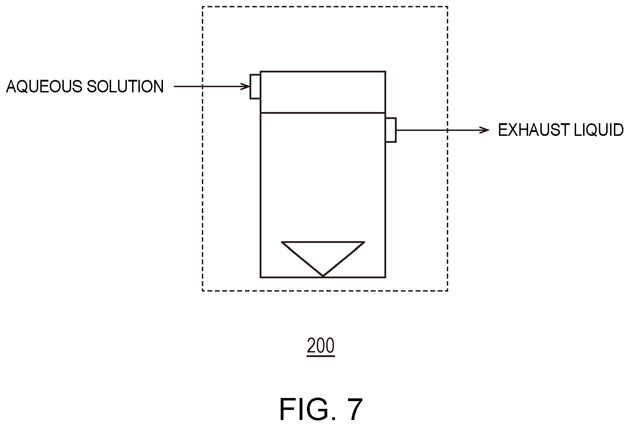

Fig. 7 is a schematic diagram illustrating a treatment apparatus according to Comparative Example 1. A treatment apparatus 200 stores activated sludge in an opened container, and introduces an acrylonitrile-containing exhaust liquid to be aerated. That is, the treatment apparatus 200 is an activated sludge waste liquid treatment apparatus using a general activated sludge process.

However, since acrylonitrile is volatile and water-insoluble, the acrylonitrile volatilizes in the aerated activated sludge and leaks to the outside.

Specifically, as shown in Fig. 9, when the amount of acrylonitrile (AN) contained in the exhaust liquid (aqueous solution) supplied to the activated sludge was about 7.5 mmol, the amount of volatilized acrylonitrile was about 4.0 mmol. After the treatment, no acrylonitrile was detected in the exhaust liquid discharged from the treatment apparatus. Only about 0.2 mmol of acrylamide and about 1.5 mmol of acrylate were detected in the exhaust liquid discharged from the treatment apparatus.

### (Comparative Examples 2 and 3)

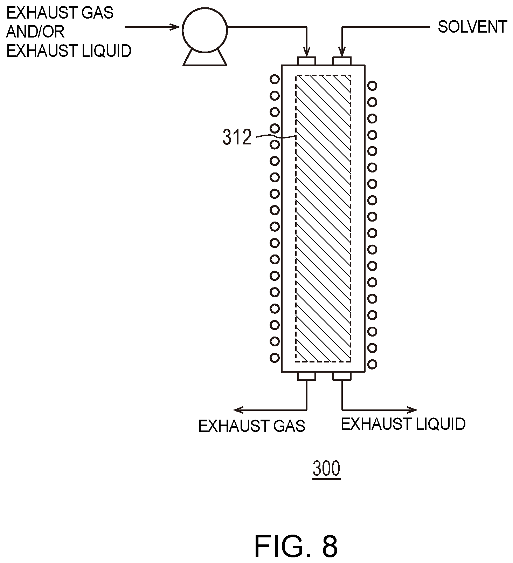

Fig. 8 is a schematic diagram illustrating a treatment apparatus according to Comparative Examples 2 and 3. A treatment apparatus 300 stores, in a casing, a catalyst 312 in which general activated sludge is immobilized on a carrier. In Comparative Example 2, an acrylonitrile-containing exhaust gas is used as a target to be treated, and a solvent is introduced into the casing of the treatment apparatus 300 to activate activated sludge. On the other hand, in Comparative Example 3, the acrylonitrile-containing exhaust liquid is a target to be treated, and the solvent is not introduced into the treatment apparatus 300. Other configurations and conditions of Comparative Examples 2 and 3 are the same.

The exhaust gas and/or exhaust liquid is introduced into the casing of the treatment apparatus 300 to contact and pass through the catalyst. The acrylonitrile in the exhaust gas and/or exhaust liquid that has come into contact with the catalyst in the casing is converted by the activated sludge to a certain extent into a metabolite downstream of acrylamide. The exhaust liquid that has passed through the casing dissolves a water-soluble metabolite downstream of acrylamide and is discharged from the drainage port. Unreacted acrylonitrile is partially dissolved in a solvent or exhausted to the outside of the casing while being contained in the exhaust gas.

A polyurethane sponge of about 1 cm square was used as a carrier of the catalyst 312 in which the activated sludge in this experiment was immobilized on a carrier. A polyurethane sponge having a bulk volume of 150 mL was put into 100 mL of activated sludge, and after shaking and stirring for 2 to 3 days, the polyurethane sponge was recovered, washed with water, and then filled in the casing as the catalyst 312.

In such a treatment apparatus 300, since the casing is sealed, the volatilization amount of acrylonitrile is almost 0. However, in activated sludge, acrylonitrile is not efficiently converted into acrylamide, and a large amount of acrylonitrile is exhausted in an unreacted state.

Specifically, in Comparative Example 2 in which the acrylonitrile-containing exhaust gas was used as a target to be treated, as shown in Fig. 9, the amount of acrylonitrile exhausted in an unreacted state was about 2.8 mmol, and acrylonitrile was not detected in the exhaust liquid discharged from the treatment apparatus. Only about 0.2 mmol of acrylamide and about 3.5 mmol of acrylate were detected.

In Comparative Example 3 in which an acrylonitrile-containing exhaust liquid was used as a target to be treated, as shown in Fig. 9, about 1.9 mmol of acrylonitrile remaining in the exhaust liquid discharged from the acrylonitrile converter 10 was detected. About 0.6 mmol of acrylamide was detected. About 2.5 mmol of acrylate was detected.

As described above, in Comparative Examples 2 and 3, the amount of volatilized or unreacted acrylonitrile is smaller and better than that in Comparative Example 1, but a large amount of acrylamide or acrylate remains in the discharged exhaust liquid, and cannot be released to the environment as it is.

### (Example 1)

An acrylonitrile-containing exhaust gas was treated according to the first embodiment illustrated in the schematic diagram of Fig. 1. The biocatalyst 12 is not activated sludge but a catalyst in which a microorganism or enzyme having nitrile hydratase activity (for example, a bacterium belonging to the genus Rhodococcus, more specifically, to Rhodococcus rhodochrous, and further specifically, to Rhodococcus rhodochrous J1 strain (Accession No.: FERM-BP-1478)) is immobilized on a carrier.

As a carrier of the biocatalyst 12 in this experiment, a polyurethane sponge of about 1 cm square was used as in Comparative Example 2. The biocatalyst 12 was prepared by the same method as in Comparative Example 2 except that 100 mL of a culture solution of a microorganism prepared by the following method was used instead of the activated sludge.

### (Culture of Microorganism Having Nitrile Hydratase Activity)

Rhodococcus rhodochrous J1 strain (Accession No.: FERM-BP-1478) was inoculated into 5 mL of LB medium and cultured in a shaking incubator under the conditions of 30°C and 120 rpm. After the proliferation was confirmed, the cells were passaged in 100 mL of a medium (pH 7.0) containing 2 wt% of glucose, 1 wt% of urea, 0.5 wt% of peptone, 0.3 wt% of yeast extract, and 0.01 wt% of cobalt chloride hexahydrate, and cultured at 30°C and 120 rpm to obtain a culture solution of a microorganism having nitrile hydratase activity.

In the experimental result with the acrylonitrile-containing exhaust gas shown in Fig. 9, the amount of acrylonitrile exhausted in an unreacted state was about 0.2 mmol, which was very small. The intermediate exhaust liquid is a solvent (solvent in the pipe P4) discharged from the acrylonitrile converter 10 in Fig. 1. Acrylonitrile was not detected in the intermediate exhaust liquid. About 7.2 mmol of acrylamide was detected. About 0.1 mmol of acrylate was detected. From this result, it can be seen that most of the acrylonitrile of the exhaust gas supplied to the acrylonitrile converter 10 was converted into acrylamide by the biocatalyst 12.

Further, the intermediate exhaust liquid containing acrylamide was supplied to the solvent treater 20 and aerated in the activated sludge, so that acrylonitrile and acrylamide were not detected in the final exhaust liquid to be discharged from the solvent treater 20. About 0.2 mmol of acrylate was detected. From this result, it can be seen that most of the acrylamide in the intermediate exhaust liquid supplied to the solvent treater 20 was converted into a substance in a metabolic pathway downstream of acrylate by the activated sludge.

### (Example 2)

An experiment was performed by the same method as in Example 1 except that the acrylonitrile-containing exhaust liquid was used as a target to be treated, and no solvent was used. Acrylonitrile remaining in the intermediate exhaust liquid was about 0.2 mmol, and the amount of acrylonitrile that could not be converted by the biocatalyst 12 was very small. About 7.2 mmol of acrylamide was detected. About 0.1 mmol of acrylate was detected. From this result, it can be seen that most of the acrylonitrile of the exhaust gas supplied to the acrylonitrile converter 10 was converted into acrylamide by the biocatalyst 12.

Further, the intermediate exhaust liquid containing acrylamide was supplied to the solvent treater 20 and aerated in the activated sludge, so that acrylonitrile and acrylamide were not detected in the final exhaust liquid to be discharged from the solvent treater 20. About 0.1 mmol of acrylate was detected. From this result, it can be seen that most of the acrylamide in the intermediate exhaust liquid supplied to the solvent treater 20 was converted into a metabolite downstream of acrylate by the activated sludge.

### (Example 3)

The biocatalyst 12 of Example 3 was prepared by the same method as in Example 1 except that granular activated carbon (AS ONE Corporation) was used as a carrier. An experiment was performed by the same method as in Example 2 except that acrylonitrile contained in the acrylonitrile-containing exhaust liquid was about 4.2 mmol. Acrylonitrile remaining in the intermediate exhaust liquid was not detected. About 4.1 mmol of acrylamide was detected. About 0.1 mmol of acrylate was detected. From this result, it can be seen that most of the acrylonitrile of the exhaust gas supplied to the acrylonitrile converter 10 was converted into acrylamide by the biocatalyst 12.

Further, the intermediate exhaust liquid containing acrylamide was supplied to the solvent treater 20 and aerated in the activated sludge, so that acrylonitrile, acrylamide, and acrylate were not detected in the final exhaust liquid to be discharged from the solvent treater 20. From this result, it can be seen that the acrylamide in the intermediate exhaust liquid supplied to the solvent treater 20 was converted into a metabolite downstream of acrylate by the activated sludge.

### (Example 4)

In Example 4, the biocatalyst 12 is a catalyst containing a microorganism and an enzyme having nitrilase activity instead of the microorganism or enzyme having nitrile hydratase activity. As a carrier, bamboo charcoal crushed to 5 to 10 mm was used. The exhaust liquid supplied to the acrylonitrile converter 10 contained 3300 ppm of acrylonitrile and was continuously supplied using a tube pump. The liquid feeding rate was 18 mL/hr. The reaction was carried out in a room kept in a range of 24 to 26°C.

In addition to the activated sludge, 0.1 g/L of potassium dihydrogen phosphate, 0.2 g/L of dipotassium hydrogen phosphate, 0.05 g/L of magnesium sulfate heptahydrate, 0.05 g/L of sodium chloride, 0.004 g/L of calcium chloride dihydrate, and 0.0005 g/L of iron sulfate heptahydrate were added to the solvent treater 20, and a decomposition reaction was performed at 30°C. Other configurations of Example 4 may be the same as any of the configurations of Examples 1 to 3.

### (Culture of Microorganism Having Nitrilase Activity)

One kind of bacteria belonging to the genus Acinetobacter, one kind of bacteria belonging to the genus Alcaligenes, and one kind of bacteria belonging to the genus Delftia were isolated from the wastewater sample of the Chiba Plant of Toray Industries, Inc. These were each inoculated into 5 mL of LB medium and cultured in a shaking incubator under the conditions of 30°C and 120 rpm. The cells were passaged in 100 mL of LB medium and cultured under the conditions of 30°C and 120 rpm to obtain a culture solution of a microorganism having nitrilase activity.

In the experimental result with the acrylonitrile-containing exhaust liquid shown in Fig. 9, acrylonitrile was not detected in the intermediate exhaust liquid. Acrylamide was also not detected in the intermediate exhaust liquid. About 13 mmol of acrylate was detected in the intermediate exhaust liquid. From this result, it can be seen that most of the acrylonitrile of the exhaust liquid supplied to the acrylonitrile converter 10 was directly converted into acrylate by the biocatalyst 12 in the acrylonitrile converter 10 of the first step. It is considered that some of the acrylate was further metabolized and used for the growth of microorganisms.

Further, the intermediate exhaust liquid containing acrylate was supplied to the solvent treater 20 and aerated in the activated sludge, so that acrylonitrile and acrylate were not detected in the final exhaust liquid to be discharged from the solvent treater 20. From this result, it can be seen that most of the acrylate in the intermediate exhaust liquid supplied to the solvent treater 20 was converted into a substance in a metabolic pathway downstream of acrylate by the activated sludge.

As described above, the exhaust gas and/or exhaust liquid treatment apparatus according to the first embodiment illustrated in the schematic diagram of Fig. 1 can efficiently convert acrylonitrile into acrylamide or acrylate in the acrylonitrile converter 10, and can efficiently convert acrylamide into a substance produced after the acrylate in the solvent treater 20, or can convert the acrylate into a harmless organic substance or inorganic substance.

Whereas some embodiments of the present invention have been described, these embodiments are merely presented as examples, and not intended to limit the scope of the invention. These embodiments can be implemented in various other forms, and various omissions, substitutions, and changes can be made without departing from the gist of the invention. These embodiments and modifications thereof are included in the scope and gist of the invention and are included in the invention described in the claims and the equivalent scope thereof.

### REFERENCE SIGNS LIST

- 1: Exhaust gas and/or exhaust liquid treatment apparatus
- 10: Acrylonitrile converter
- 11: Casing
- 12: Biocatalyst
- 13: Pump
- In1: First introduction port
- In2: Second introduction port
- Out1: Exhaust port
- Out2: Drainage port
- Stmp: Temperature sensor
- Ctmp: Temperature controller
- Atmp: Temperature adjuster
- SpH: pH sensor
- CpH: pH controller
- TpH: pH adjuster introduction unit
- 20: Solvent treater
- 21: Container
- 22: Gas supply unit
- In3: Third introduction port
- Out3: Drainage port

## Claims

1. An exhaust gas or exhaust liquid treatment method comprising:
a first step of bringing acrylonitrile in an exhaust gas or exhaust liquid into contact with a biocatalyst and a solvent activating a biocatalyst to convert the acrylonitrile into acrylamide and/or acrylate; and
a second step of decomposing the acrylamide and/or acrylate.

2. The exhaust gas or exhaust liquid treatment apparatus according to claim 1, wherein the biocatalyst is one or two or more biocatalysts selected from the group consisting of a microorganism and an enzyme having nitrile hydratase activity and a microorganism and an enzyme having nitrilase activity.

3. The exhaust gas or exhaust liquid treatment apparatus according to claim 2, wherein the microorganism having nitrile hydratase activity is one or two or more microorganisms selected from the genus Rhodococcus and the genus Comamonas.

4. The exhaust gas or exhaust liquid treatment apparatus according to claim 2, wherein the microorganism having nitrile hydratase activity is Rhodococcus rhodochrous.

5. The exhaust gas or exhaust liquid treatment apparatus according to claim 2, wherein the microorganism having nitrilase activity is one or two or more microorganisms selected from the genus Rhodococcus, the genus Acinetobacter, the genus Alcaligenes, and the genus Delftia.

6. The exhaust gas or exhaust liquid treatment method according to claim 1, wherein the first step and the second step are steps independent from each other.

7. The exhaust gas or exhaust liquid treatment method according to claim 1, wherein a reaction temperature in the first step is 20 to 30°C.

8. The exhaust gas or exhaust liquid treatment method according to claim 1, wherein a pH of the acrylonitrile, the biocatalyst, and the solvent in the first step is a pH of 6 to 8.

9. The exhaust gas or exhaust liquid treatment method according to claim 1, wherein an air flow rate to the acrylonitrile, the biocatalyst, and the solvent in the first step is 5 vvm or less.

10. The exhaust gas or exhaust liquid treatment method according to claim 1, wherein a concentration of acrylonitrile in the exhaust gas or the exhaust liquid is 10000 ppm.

11. The exhaust gas or exhaust liquid treatment method according to claim 1, wherein the first step is a step of causing the acrylonitrile and the solvent activating a biocatalyst to pass through the biocatalyst in a direction of gravity to convert the acrylonitrile and the solvent into a solution containing acrylamide and/or acrylate.

12. An exhaust gas or exhaust liquid treatment apparatus comprising:
an acrylonitrile converter including a casing that houses an immobilized biocatalyst, an acrylonitrile introduction port through which an exhaust gas or exhaust liquid containing acrylonitrile is introduced into the casing, a solvent introduction port through which a solvent activating the immobilized biocatalyst is introduced, and a drainage port through which a solution containing acrylamide and/or acrylate produced from acrylonitrile by the immobilized biocatalyst is discharged from the casing; and
a treater treating the solution containing the acrylamide and/or the acrylate discharged from the acrylonitrile converter.

13. The exhaust gas or exhaust liquid treatment apparatus according to claim 12, wherein the solvent introduction port is the acrylonitrile introduction port.

14. The exhaust gas or exhaust liquid treatment apparatus according to claim 12, wherein the acrylonitrile converter is a fixed-bed catalyst reactor in which the casing is filled with the immobilized biocatalyst.

15. The exhaust gas or exhaust liquid treatment apparatus according to claim 12, wherein the acrylonitrile introduction port and the solvent introduction port are installed at an upper end of the casing.

16. The exhaust gas or exhaust liquid treatment apparatus according to claim 12, wherein the acrylonitrile converter includes an exhaust port through which the exhaust gas that has passed through the immobilized biocatalyst is exhausted from the casing.

17. The exhaust gas or exhaust liquid treatment apparatus according to claim 12, wherein a carrier of the immobilized biocatalyst is activated carbon or wood-based char.

18. The exhaust gas or exhaust liquid treatment apparatus according to claim 12, wherein the acrylonitrile converter further includes:
a temperature sensor detecting a temperature of the immobilized biocatalyst;
a temperature adjuster adjusting the temperature of the immobilized biocatalyst; and
a temperature controller controlling the temperature adjuster based on a temperature measurement value from the temperature sensor.

19. The exhaust gas or exhaust liquid treatment apparatus according to claim 12, wherein the acrylonitrile converter further includes:
a pH sensor detecting a pH of the immobilized biocatalyst;
a pH adjuster introduction unit introducing a pH adjuster for adjusting the pH of the immobilized biocatalyst into the solvent; and
a pH controller controlling the pH adjuster introduction unit based on a pH measurement value from the pH sensor.

20. The exhaust gas or exhaust liquid treatment apparatus according to claim 12, wherein the treater stores activated sludge for treating acrylamide and/or acrylate.

21. The exhaust gas or exhaust liquid treatment apparatus according to claim 20, wherein the activated sludge has amidase activity.
